# EUROPEAN PATENT APPLICATION

(11) **EP 1 739 621 A1**
(43) Date of publication of application: **03.01.2007**
(21) Application number: 06253444.1
(22) Date of filing: 30.06.2006
(51) Int. Cl.: G06T 5/00

(54) **Image processing**

(30) Priority: 01.07.2005 GB 0513532
(71) Applicant: University College Cardiff Consultants, Ltd., Cardiff South Glamorgan CF24 0DE (GB)
(72) Inventor: Toumi, Hechmi, University College Cardiff, Cardiff CF10 2US (GB)
(74) Representative: Gillard, Matthew Paul

(57) **Abstract**

A method of processing an image containing at least one object boundary, the method comprising producing a contour map of the image in which contours divide the image into zones and merging zones if their statistical properties of their pixels sufficiently match those of the pixels expected of an object that is known or thought to be present in the image. The invention extends to corresponding apparatus. The image may be a medical image, for example an X-ray of a joint.

## Description

The invention relates to image processing techniques that can be used to enhance medical images such as X-ray pictures and MRI pictures. Of course, the image processing techniques provided by the invention are applicable to other types of picture.

According to one aspect, the invention provides a method of processing a medical image, the method comprising rendering the image into a contour map and modifying the arrangement of the contours under the guidance of histological data so that the contours resolve into the boundaries between different physical structures in the image.

The invention also consists in apparatus for processing a medical image, the apparatus comprising means for rendering the image into a contour map and means for modifying the arrangement of the contours under the guidance of histological data so that the contours resolve into the boundaries between different physical structures in the image.

By processing an image in this way, it is possible to bring out details of the image in a meaningful way. Typically, X-ray pictures do not provide meaningful information about soft tissue such as tendon, ligament and cartilage. In particular, by processing an X-ray picture in a manner according to the invention, it is possible to recover meaningful information about the soft tissue of the kind just mentioned. This is of particular benefit in the non-invasive diagnosis of joint, tendon and ligament problems and tumours.

According to another aspect, the invention provides a method of processing an image containing at least one object boundary, the method comprising producing a contour map of the image in which contours divide the image into zones and merging zones if the statistical properties of their pixels match those of the pixels expected of an object that is known or thought to be present in the image.

The invention also consists in apparatus for processing an image containing at least one object boundary, the apparatus comprising means for producing a contour map in which contours divide the image into zones and means for merging zones if the statistical properties of their pixels match those of pixels expected of an object that is known or thought to be present in the image.

Typically, although not exclusively, the image processed by the invention is a medical image, such as an X-ray or an MRI picture. The object whose expected pixel properties are used to guide the merging of image zones may or may not be a homogenous object. For example, such an object could comprise a piece of articular cartilage that itself comprises deep, transitional and superficial layers.

The invention also consists in a method of making a diagnosis about the condition of a human or an animal at least partly on the basis of a medical image that has been processed using the techniques prescribed by the invention.

By way of example only, certain embodiments of the invention will now be described with reference to the accompanying drawings in which:
Figure 1 is a block diagram of an X-ray machine connected to a personal computer;
Figure 2 is a schematic illustration of an X-ray picture of a bone fragment;
Figure 3 is a flow chart of steps for analysing a part of the image of Figure 2;
Figure 4 illustrates the selection of a region of interest in the X-ray image of Figure 2;
Figure 5 illustrates an enlargement of the region of interest selected in Figure 4;
Figure 6 illustrates a contour map that has been derived from the section of the X-ray that is shown in Figure 5;
Figure 7 illustrates a contour map of a histological image of the region of interest shown in Figures 5 and 6;
Figure 8 illustrates the selection of further regions of interest in the X-ray picture of Figure 2;
Figure 9 is an X-ray of a knee joint;
Figure 10 is a region of interest within the X-ray of Figure 9 that has been enhanced using the techniques of the invention;
Figure 11 is an enlargement of a region of Figure 10;
Figure 12 is an enlargement of another region of Figure 10;
Figure 13 is an image of the tibial plateau of the joint shown in Figure 9;
Figure 14 is an X-ray of a fractured limb;
Figure 15 is a region of interest within the X-ray of Figure 14 that has been enhanced using the techniques of the present invention; and
Figure 16 is an enlargement of a portion of the image of Figure 15.

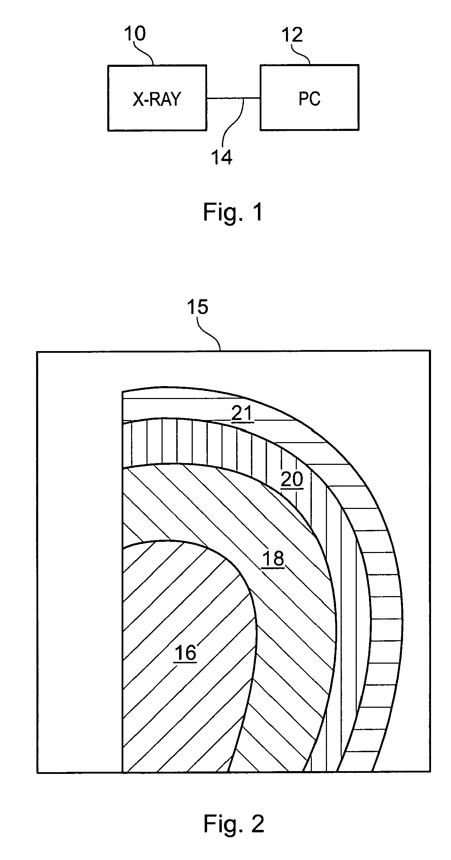

Figure 1 shows a medical X-ray machine 10 connected to a PC (personal computer) 12. X-ray pictures taken by machine 10 are delivered over connection 14 to the PC 12 for processing.

We will now consider the case where X-ray machine 10 is used to analyse a fragment of a bone from a joint of a cadaver, the fragment being covered with articular cartilage. Figure 2 shows a picture 15 of the bone fragment taken by the machine 10. The picture 15 contains four differently shaded areas 16, 18, 20 and 21. Area 16 represents bone and areas 18, 20 and 21 represent the deep, transitional and superficial layers, respectively, of the articular cartilage.

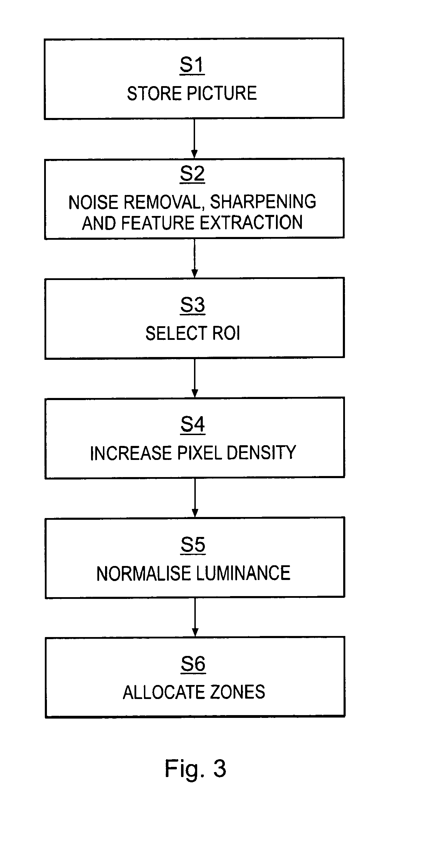

The unshaded areas in the picture 15 represent the free space around the bone fragment in the field of view of the X-ray machine 10. For clarity's sake, the areas 16, 18, 20 and 21 are shown clearly delimited from one another in Figure 2 although one skilled in the art will realise immediately that, in reality, areas 16, 18, 20 and 21 blur into one another. The PC 12 processes the X-ray picture 15 using the procedure set out in the flow chart of Figure 3.

In step S1, the data representing picture 15 is received by the PC 12 from machine 10 and is stored as a two dimensional array of pixels.

In step S2, a succession of finite impulse response (FIR) filters are applied to picture 15 for noise removal, image sharpening and feature extraction. Appropriate filtering algorithms to achieve these goals will be readily apparent to one skilled in the art.

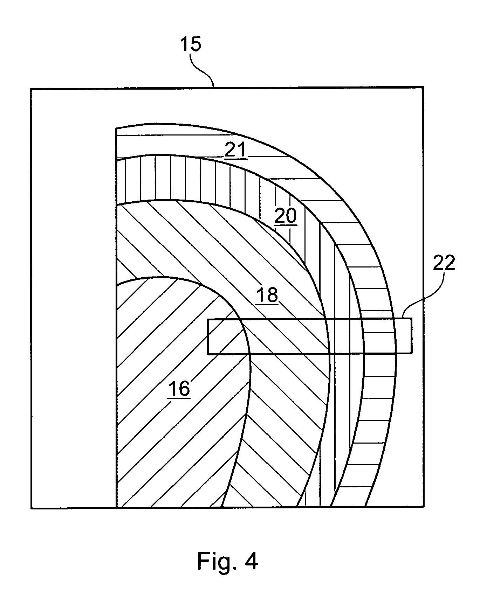

In step S3, a region of interest (ROI) 22 is selected for the subsequent processing stages. The ROI 22 is selected to include parts of the bone 16, the three articular cartilage layers 18, 20, and 21 and the background, as shown in Figure 4.

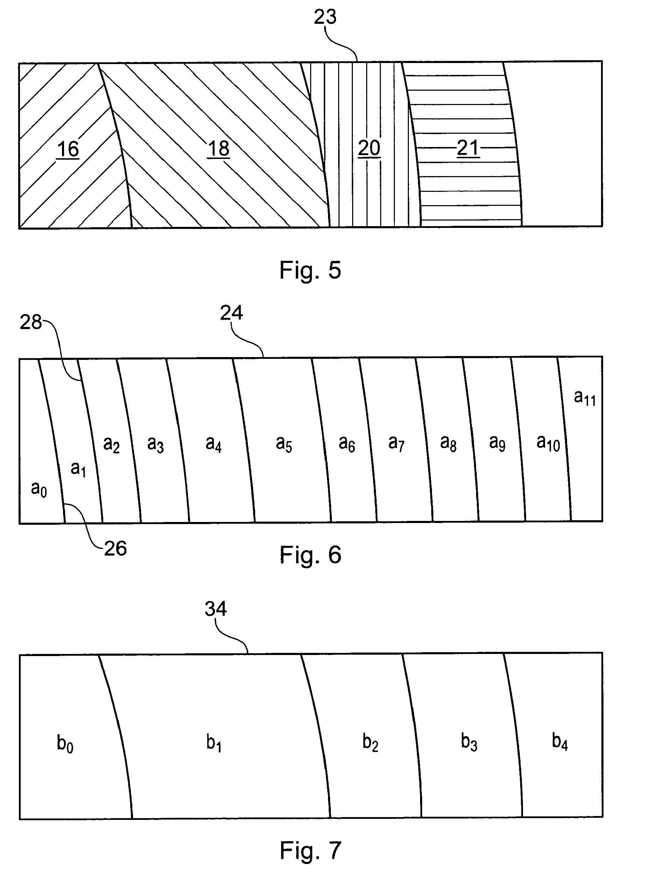

Figure 5 shows an enlargement of the ROI 22. The part of the picture bounded by the ROI 22 will henceforth be referred to as an image under analysis (IUA) 23 and is treated as a separate two dimensional pixel array in its own right in steps S4 to S6 that follow.

In step S4, the pixel density of the IUA 23 is increased by either or both of a Laplacian pyramid filter and a Gaussian pyramid filter. Appropriate algorithms for implementing such filters will be readily apparent to one skilled in the art. The effect of these filters is to interpolate within the IUA 23 thus increasing the density of pixels within the IUA 23. The increase in pixel density employed is typically a factor in the range 6 to 12.

In step S5, the characteristics of the pixels in the IUA 23 other than luminance are discarded. Next, the maximum and minimum luminance values of the pixels within the IUA 23 are detected and used to calculate the luminance range for the IUA. The luminance range is then mapped onto a range of values extending from 0 to 255 such that the lowest luminance value in the IUA 23 is replaced with 0, the highest luminance value is replaced with 255 and the intervening luminance values are replaced with proportionate values in the range 0 to 255. Thus the IUA 23 is converted into a normalised luminance array (NLA). For the purpose of displaying image data arrays of this type on its screen (not shown), the PC 12 is configured to display each value in the 0 to 255 range as a different colour in a graduated spectrum.

In step S6, the IUA 23 is analysed with the aim of detecting the boundaries of its bone and articular cartilage zones. First, the NLA undergoes contour filtering to create, as shown in Figure 6, a contour map 24 of the NLA having contour lines representing the magnitude of the normalised luminance values assigned to the pixel in the NLA. An appropriate algorithm for conducting the contour filtering will be readily apparent to one skilled in the art.

The map 24 is divided into zones by its contours. For example, contour lines 26 and 28 define zones a₀ and a₁ as seen in Figure 6. The quantity of contours allocated to the map 24 is deliberately chosen to divide the map 24 into a number of regions that is greater than the number of physically distinct zones that are known to be present in the ROI 22. In the present case, the ROI 22 is known to contain five different zones (of bone, deep, transitional and superficial articular cartilage and background space, respectively) so eleven contours are used in map 24 to divide the map into twelve zones a₀, a₁, a₂, ... a₁₁ (from left to right in Figure 6). Next, the zones in the contour map 24 are considered for merging with the aim of reducing the number of zones to the number known to be present in the ROI 22, i.e. down to five. The amalgamation of the contour map zones is guided by histological data as will now be explained.

A histological image 34, as shown in Figure 7, of the ROI 22 is imported to the PC 12. The pixels in the histological image 34 have differing luminance values on account of the staining applied in the histology.

A contour filter is applied to the histological image 34 to detect the boundaries of the five zones that are known to be present in the ROI 22. Thus, the histological image is divided into five zones b₀, b₁, b₂, b₃ and b₄ containing bone, deep articular cartilage, transitional articular cartilage, superficial articular cartilage and background, respectively. Next, the zones a₀ to a₁₁ are allotted to pairs of adjacent zones, i.e. a₀ with a₁, a₂ with a₃, a₄ with as and so on. Consideration is then given to merging the zones within the pairs to reduce the number of zones present in contour map 24. To explain this procedure, we will now consider the pair of zones a₀ and a₁.

First, the standard deviation of the normalised luminance values in the part of the NLA covered by zones a₀ and a₁ is calculated. That value is then compared with the standard deviation of the luminance values of the pixels in zone b₀ of the histological image. If the two standard deviation values are within 5% of each other, then the comparison is considered positive. Next, rank-order correlation and Kolmogorov-Smirnov tests are used to produce a correlation coefficient between, on the one hand, the normalised luminance values of the combined pixel population of a₀ and a₁ and, on the other hand, the luminance values of the pixel population of b₀. If the correlation coefficient is ≥ 0.95 , then the correlation comparison is considered positive. If both the correlation and the standard deviation comparisons are positive, then the two zones a₀ and a₁ are merged into a single zone a₀₊₁.

Following completion of the merger test on a₀ and a₁, the merger test is performed on a₂ and a₃. If a₀ and a₁ were allowed to merge, then the combined population of a₂ and a₃ is tested against the population of b₀ or otherwise against that of b₁. In this manner, the procedure progresses through the series of zones b₀ to b₄ when testing the pairs a₂ₘ, a₂ₘ₊₁ for merger.

Once the merger test has been performed on all of the pairs a₂ₘ, a₂ₘ₊₁ for m=0 to 5, a check is made to determine if the number of zones in the map 24 is still greater than five. If the number of zones in the map 24 is found to be greater than five, then the surviving zones are re-examined to determine if any zones within pairs of adjacent zones can be merged. This iterative procedure continues until the number of zones in the map 24 reduces to five, at which point the boundaries of the four surviving zones should, from left to right in the map, accurately reflect the contours of the bone, deep articular cartilage, transitional articular cartilage, superficial articular cartilage and background regions, respectively.

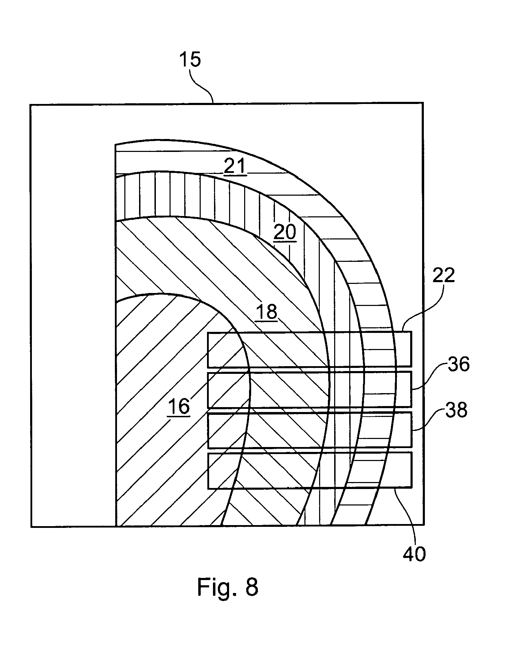

As shown in Figure 8, further ROIs, e.g. 36, 38 and 40 can be processed in the manner explained above in order to build up information about a larger part of the bone fragment.

It will be apparent to one skilled in the art that, when the process explained by reference to Figures 2 to 8 is used to enhance an X-ray of a whole joint in a living patient, rather than of an isolated bone fragment, the improved imaging of the associated soft tissue facilitates the evaluation of the condition of the joint. Likewise, the technique can be applied to images of all other soft tissues, notably tendons and ligaments, muscles, intervertebral discs, blood vessels, brain, spinal cord, nerves, breast and prostate gland. It could also be applied to visualise tumours anywhere in the body (particularly in bone) and to visualise the repair of bone fractures and monitor changes in cataracts. Thus, the technique is not just limited to cartilage on bones, which is merely the scenario chosen for the purpose of the embodiment described above. It is likely to be of general applicability to any soft tissue in the body.

It will be apparent that the invention can be delivered as a software package (on a CD, for example) for installation on any compatible computer (or other data processing equipment) that is capable of receiving digital images for analysis. Such software will typically be tailored for the analysis of one or more particular image types and will therefore contain knowledge of the expected statistical properties of the objects that are to be expected in these image types in order to guide the decisions on the merger of zones in IUAs. That is to say, the software will carry, for the target image types, the equivalent of the expected statistical properties of the b₀₋b₄ zones of the cadaverous sample featured in the embodiment described above. By way of a more concrete example, if the software package is tailored for the analysis of breast tumours and knee joint analysis, then the package is imbued with the expected statistical properties of objects that would be expected to be found in breast and knee joint images.

Some examples of image details revealed through application of the present invention to X-ray images will now be provided.

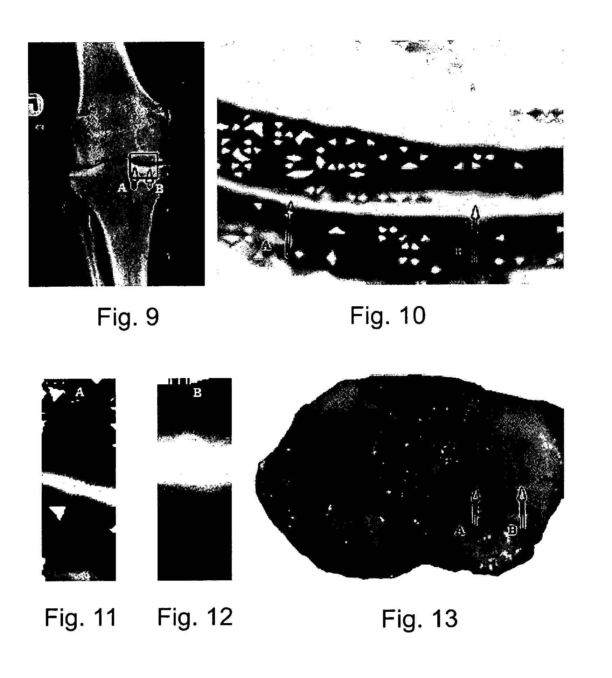

Figure 9 is an X-ray picture of a knee joint that is provided for enhancement using the present invention. An ROI for enhancement by the present invention is demarcated by the black frame overlaid on the figure. The result of enhancing the ROI by applying the processing techniques of the invention is shown in Figure 10. In that figure, the image zone corresponding to the hyaline cartilage appears as a light coloured band between two darker coloured zones. It should be noted that the image enhancement techniques of the invention have revealed that the hyaline cartilage has degenerated in the region indicated by arrow A as compared to the region indicated by arrow B. The parts of the enhanced ROI of Figure 10 as indicated by arrows A and B are shown magnified in Figures 11 and 12, respectively. The joint shown in Figure 9 was removed and an examination of the tibial plateau, as shown in Figure 13, revealed that there was indeed degeneration of the tibial plateau at the point corresponding to the section imaged in Figure 11.

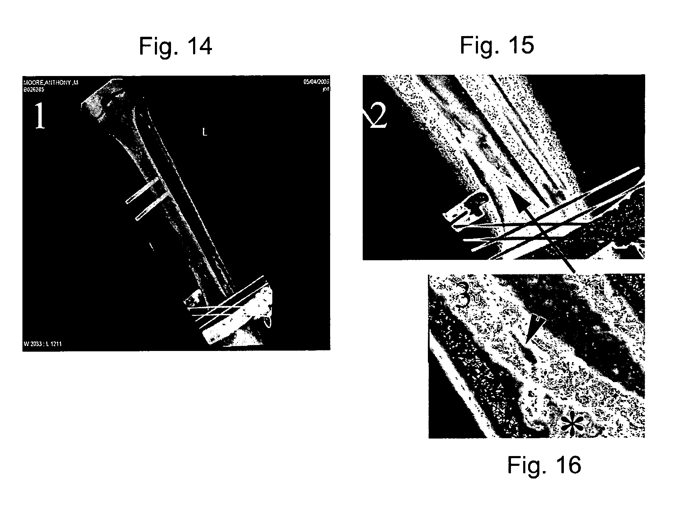

Figure 14 is an X-ray image of a fractured limb. Figure 15 shows the result of enhancing an ROI of the X-ray using the techniques of the present invention. The area within Figure 15 indicated by an arrow is shown magnified in Figure 16. The arrow-head and star symbols denote respectively regions of non-regenerated and regenerated bone that can be visualised using the present invention.

## Claims

1. A method of processing a medical image, the method comprising rendering the image into a contour map and modifying the arrangement of the contours under the guidance of histological data so that the contours resolve into the boundaries between different physical structures in the image.

2. A method according to claim 1, wherein the rendering of the image into a contour map produces a contour map of the image in which contours divide the image into zones and modifying the contour arrangement comprises merging zones if the statistical properties of their pixels sufficiently match those of the pixels expected of an object that is known or thought to be present in the image.

3. A method of processing a medical image containing at least one object boundary, the method comprising producing a contour map of the image in which contours divide the image into zones and merging zones if the statistical properties of their pixels sufficiently match those of pixels expected of an object that is known or thought to be present in the image.

4. A method according to claim 2 or 3, wherein the statistical properties comprise the standard deviation of the luminance values of, on the one hand, the pixels expected of the object and, on the other hand, the pixels of zones proffered for merger.

5. A method according to claim 2, 3 or 4, wherein the assessment of the degree of match of the statistical properties comprises correlating the luminance values of the pixels expected of the object with the luminance values of the pixels of zones that are proffered for merger.

6. A method of processing an image containing at least one object boundary, the method comprising producing a contour map of the image in which contours divide the image into zones and merging zones if the statistical properties of their pixels sufficiently match those of the pixels expected of an object that is known or thought to be present in the image.

7. A method according to any one of the preceding claims, further comprising filtering the image before producing from it a contour map, wherein the filtering process comprises one or more of noise removal filtering, feature extraction filtering or edge sharpening filtering.

8. A method according to any one of the preceding claims, wherein said image is one of an X-ray picture and an MRI picture.

9. A method according to any one of the preceding claims, wherein said image is of or is of part of a joint.

10. A method of processing an image, comprising discerning several regions of interest within the image and, for each of a plurality of said regions, processing the region according to the method of any one of the preceding claims.

11. Apparatus for processing a medical image, the apparatus comprising means for rendering the image into a contour map and means for modifying the arrangement of the contours under the guidance of histological data so that the contours resolve into the boundaries between different physical structures in the image.

12. Apparatus according to claim 11, wherein the rendering means produces a contour map of the image in which contours divide the image into zones and the modifying means merges zones if the statistical properties of their pixels sufficiently match those of the pixels expected of an object that is known or thought to be present in the image.

13. Apparatus for processing a medical image containing at least one object boundary, the apparatus comprising means for producing a contour map of the image in which contours divide the image into zones and means for merging zones if the statistical properties of their pixels sufficiently match those of pixels expected of an object that is known or thought to be present in the image.

14. Apparatus according to claim 12 or 13, wherein the statistical properties comprise the standard deviation of the luminance values of, on the one hand, the pixels expected of the object and, on the other hand, the pixels of zones proffered for merger.

15. Apparatus according to claim 12, 13 or 14, wherein the modifying means is arranged to correlate the luminance values of the pixels expected of the object with the luminance values of the pixels of zones that are proffered for merger.

16. Apparatus for processing an image containing at least one object boundary, the apparatus comprising means for producing a contour map of the image in which contours divide the image into zones and means for merging zones if the statistical properties of their pixels sufficiently match those of the pixels expected of an object that is known or thought to be present in the image.

17. Apparatus according to any one of claims 11 to 16, further comprising means for filtering the image before producing from it a contour map, wherein the filtering means is arranged to apply one or more of noise removal filtering, feature extraction filtering or edge sharpening filtering.

18. A program for causing data processing equipment to perform a method according to any one of claims 1 to 10.

19. A method of diagnosing the condition of a biological entity, comprising use of an image that has been processed by the method of any one of claims 1 to 10.
